# EUROPÄISCHE PATENTSCHRIFT

(11) **EP 1 382 967 B1**
(45) Veröffentlichungstag und Bekanntmachung des Hinweises auf die Patenterteilung: **11.01.2006**
(21) Anmeldenummer: 03012363.2
(22) Anmeldetag: 30.05.2003
(51) Int. Cl.: G01N 33/543

(54) **Verfahren und Diagnostikum zum qualitativen oder quantitativen Nachweis von Antikörpern und zur Bestimmung von deren Avidität**
Process and reagent for quantitatively and qualitatively detecting antibodies and for evaluating their avidity
Procédé et agent diagnostique pour déterminer quantitativement et qualitativement des anticorps et pour évaluer leur avidité

(30) Priorität: 16.07.2002 DE 10232203
(43) Veröffentlichungstag der Anmeldung: 21.01.2004
(73) Patentinhaber: Institut Virion/Serion GmbH, 97072 Würzburg (DE)
(72) Erfinder: Hermann, Gerhard, Dr., 97072 Würzburg (DE)
(74) Vertreter: Laufhütte, Dieter

(56) Entgegenhaltungen:
- EP-A- 0 875 761
- US-A- 5 679 537
- MINEO J R ET AL: "Antibodies to Toxoplasma gondii major surface protein (SAG-1, P30) inhibit infection of host cells and are produced in murine intestine after peroral infection" JOURNAL OF IMMUNOLOGY, Bd. 150, Nr. 9, 1993, Seiten 3951-3964, XP002267581 ISSN: 0022-1767
- LEE JAE KOO ET AL: "Development of an ELISA for the detection of the residues of the insecticide imidacloprid in agricultural and environmental samples" JOURNAL OF AGRICULTURAL AND FOOD CHEMISTRY, Bd. 49, Nr. 5, Mai 2001 (2001-05), Seiten 2159-2167, XP001157354 ISSN: 0021-8561
- IBARRA NEYSI ET AL: "Comparison of different elution conditions for the immunopurification of recombinant hepatitis B surface antigen" JOURNAL OF CHROMATOGRAPHY B, Bd. 735, Nr. 2, 10. Dezember 1999 (1999-12-10), Seiten 271-277, XP002267582 ISSN: 0378-4347
- BEN-ATIA I ET AL: "Preparation of Recombinant Gilthead Seabream (Sparus aurata) Growth Hormone and Its Use for Stimulation of Larvae Growth by Oral Administration" GENERAL AND COMPARATIVE ENDOCRINOLOGY, ACADEMIC PRESS, US, Bd. 113, 1999, Seiten 155-164, XP002954054 ISSN: 0016-6480
- CAUGHEY DOROTHY J ET AL: "Fractionation of polyclonal antibodies to fragments of a neuroreceptor using three increasingly chaotropic solvents" JOURNAL OF CHROMATOGRAPHY B, Bd. 728, Nr. 1, 14. Mai 1999 (1999-05-14), Seiten 49-57, XP004166187 ISSN: 0378-4347

## Beschreibung

Die Erfindung betrifft ein Verfahren zum qualitativen oder quantitativen Nachweis von Antikörpern und zur Bestimmung von deren Avidität. Dadurch wird die Diagnose der frühen Phase viraler, bakterieller oder parasitärer Infektionen bzw. eine Differenzierung zwischen Erstinfektion oder Reinfektion ermöglicht.

Die Infektionsserologie zur Bestimmung von Antikörpern im Blut, bzw. Serum, Plasma oder Liquor zur Absicherung von klinischen Befunden hat sich seit vielen Jahren erfolgreich etabliert. Wichtig ist dabei oft die Bestimmung bzw. Rückverfolgbarkeit des Infektionszeitpunktes und die Unterscheidung zwischen erstmaliger Infektion mit einem Erreger und einer Reinfektion. Dazu wurden in der Vergangenheit die IgG-Antikörper als Zeichen einer zurückliegenden Infektion (serologische Narbe) angesehen, während vorliegende IgM-Antikörper als Hinweis auf eine gerade ablaufende oder kurz zurückliegende Infektion interpretiert wurden. Da jedoch IgM-Antikörper teilweise lange persistieren, durch polyklonale Stimulierung unspezifisch auftreten und auch bei einer Reinfektion gebildet werden können, sind auf diesem Wege keine absolut sicheren Diagnosen möglich. Hier kann die Bestimmung der Avidität der IgG-Antikörper sehr wichtige zusätzliche Informationen liefern, da die Avidität der Antikörper im Laufe der immunologischen Auseinandersetzung des Organismus mit dem Erreger zunehmend höher wird und damit Rückschlüsse auf den Infektionszeitpunkt zuläßt. Aviditätsteste werden in der Literatur für die Anwendung bei verschiedenen Erregern häufig beschrieben, z. B. K. Hedman et al. (1989), J. Med. Virol. 27:288-292; H.I.J. Thomas et al. (1988), Epidem. Inf. 101:591-598: J. Schubert et al. (1996) 20:713-717 usw. Als denaturierende Substanzen werden dabei Hamstoff, Diethylamin, Guanidin und Thiocyanat genannt. Die in der Literatur beschriebenen Verfahren besitzen leider große Nachteile, z. B. aufgrund technischer Probleme, die sich aus der erforderlichen sehr hohen Konzentration der denaturierenden Substanzen ergeben oder aufgrund anderer unangenehmer Eigenschaften dieser Stoffe. Wird mit geringeren Konzentrationen gearbeitet, ist der Aussagewert der Methode eingeschränkt, da dann nicht mehr sicher zwischen hoch- und niedrigavid unterschieden werden kann.

Die Hamstoffkonzentration kann gesenkt werden, wenn zum Aviditätsreagenz Wasserstoffperoxid beigemischt wird (siehe Patent DE 197 18 361 A1). Nachteil dieses Verfahrens ist jedoch, dass die denaturierende Substanz dann stark ätzend wirkt und nur unter Verwendung von Körperschutzmitteln angewendet werden kann.

Gegenstand der hier vorliegenden Erfindung ist die Entdeckung, dass die oben genannten Probleme dadurch gelöst werden können, dass die denaturierenden Substanzen auf jeweils definierte pH-Werte eingestellt werden und dadurch schon bei wesentlich geringeren, d. h. technisch gut anwendbaren Konzentrationen eine gleiche und sogar bessere Diskriminierung im Aviditätstest zwischen niedrig- und hochaviden Seren ermöglichen. Dabei liegt der geeignete pH-Wert überraschender Weise nicht im dem für immunologische Teste üblichen optimalen pH-Bereich der Reagenzien zur Ermöglichung der immunologischen Reaktionen von etwa 5,0 bis 9,0 (nachfolgend immer "unüblicher pH-Wert" genannt), sondern im darunter und/oder darüber liegenden pH-Bereich.

Die vorliegende Erfindung betrifft außerdem die Verfahren zur Bestimmung der Avidität eines Antikörpers, worin der Antikörper in einem ersten und einem zweiten Ansatz unabhängig voneinander mit einem Antigen, gegen welches der Antikörper gerichtet ist, in Kontakt gebracht wird, welches Immunkomplexe mit Antikörpern niedriger Avidität stabilisiert, während Immunkomplexe mit Antikörpern höherer Avidität weitgehend erhalten bleiben, und worin das Ausmaß der Bindung des Antikörpers mit dem Antigen in beiden Proben unabhängig voneinander nach einem dem Fachmann bekannten Verfahren bestimmt wird, und wobei sich die Avidität des Antikörpers aus dem Verhältnis des Ausmaßes der Antigen-Antikörperbindungen des ersten und des zweiten Ansatzes ergibt, dadurch gekennzeichnet, dass das proteindenaturierende Agenz auf einen unüblichen pH-Wert eingestellt wurde.

Bevorzugt werden Verfahren, in denen das Antigen in festphasengebundener Form mit dem Antikörper in Kontakt gebracht und anschließend mit einem proteindenaturierenden Agenz gewaschen wird, welches auf einen unüblichen pH-Wert eingestellt wurde. Beispielsweise zählen der Enzymimmunoassay, Radioimmuntest, Westem Blot oder Immunfluoreszenztest zu solchen Verfahren. Aber auch andere Teste, denen das Prinzip einer Antigen-Antikörperreaktion eigen sind, fallen unter die Patentansprüche, wenn sie erfindungsgemäß mit einem proteindenaturierenden Agenz, das auf einen unüblichen pH-Wert eingestellt wurde, durchgeführt werden sollen.

Erfindungsgemäß sind die oben genannten Verfahren insbesondere zum Nachweis diagnostisch relevanter Antikörper in Körperflüssigkeiten geeignet. Solche Antikörper können z. B. gegen Viren, Bakterien oder Parasiten gerichtet sein, wie EBV, Rötelnvirus, CMV, Parvovirus B19, VZV, HSV, Toxoplasma gondii oder auch jeden anderen Erreger, wenn die Bestimmung der Avidität der vorhandenen Antikörper sinnvoll erscheint.

Die erfindungsgemäße Aviditätstestung kann vorteilhafterweise mit kommerziell erhältlichen Immunoassays (z.B. ELISA) teil- oder vollautomatisch durchgeführt werden.

Die erfindungsgemäße Aviditätstestung ist dadurch gekennzeichnet, dass sie eine sehr gute Diskriminierung zwischen Antikörpern frischer und zurückliegender Infektionen ermöglicht. Die Unterscheidung zwischen Erst- und Reinfektionen ist dadurch möglich, wie sie z. B. bei Röteln, CMV, EBV, HSV oder Toxoplasma gondii besonders für Schwangere gefordert wird.

Die Herstellung des proteindenaturierenden Agens, das auf einen unüblichen pH-Wert eingestellt ist, erfolgt nach Verfahren, die allgemein dem Fachmann bekannt sind z. B. durch Hinzufügen einer Menge geeigneter Säure, Lauge oder saurer oder alkalischer Salze zu dem jeweils vorliegenden proteindenaturierenden Agenz. Dabei können auch kommerziell erhältliche Waschlösungen, spezielle Pufferlösungen oder Probenverdünnungspuffer zum Einsatz kommen.

Mit dem oben genannten Aviditätsreagenz mit unüblichen pH-Wert wird nach der jeweils gängigen Testdurchführung ein Immunoassay, z. B. ein ELISA durchgeführt. Dabei wird zusätzlich nach der Probeninkubation das erfindungsgemäße Aviditätsreagenz mit unüblichem pH-Wert eine jeweils optimale Zeitdauer auf die Antigen-Antikörperkomplexe einwirken gelassen. Jede Probe wird sowohl in einem Testansatz mit Aviditätsreagenz als auch in einem zweiten mit einer neutralen Vergleichssubstanz (z. B. Waschlösung mit üblichem pH-Wert) einmal oder mehrfach behandelt und aus dem Verhältnis der Meßsignale der Aviditätskoeffizient ermittelt.

In den nachfolgenden Beispielen fanden SERION ELISA *classic der* Würzburger Firma Institut Virion-Serion GmbH Verwendung, was nicht bedeutet, dass die erfindungsgemäßen Aviditätsreagenzien mit unüblichen pH-Werten auf die Teste dieses Herstellers beschränkt bleiben.

Weitere Einzelheiten und Vorteile der Erfindung werden anhand der folgenden Beispiele näher erläutert:

### Beispiel 1

Die Wirkung eines unüblichen pH-Wertes auf die Aviditätsbestimmung der Seren von Patienten mit frischer oder lang zurückliegender Infektion durch das Röteln Virus mittels ELISA sollte unter Verwendung des bekannten proteindenaturierenden Agenz Harnstoff in einem Waschschritt nachgewiesen werden. Dabei betrug die Konzentration dieser Substanz 4 Mol. Die Testung erfolgte mit dem SERION ELISA *classic* Röteln IgG.

### Ergebnis siehe Tabelle 1:

Die 4 molare Hamstofflösung bewirkt im neutralen pH-Bereich (bis pH-Wert 5,0) keine gute Diskriminierung zwischen hochaviden und niedrigaviden Seren. Wird der pH-Wert auf einen Bereich zwischen 3 und 4 eingestellt, bewirkt 4 molarer Harnstoff eine sehr gute Unterscheidung zwischen hochaviden und niedrigaviden Seren bei Probanden mit einer Immunität gegen Röteln.

### Beispiel 2

Die Wirkung eines zweiten unüblichen pH-Wertes auf die Aviditätsbestimmung der Seren von Patienten mit frischer oder lang zurückliegender Infektion durch das Röteln Virus mittels ELISA sollte unter Verwendung des bekannten proteindenaturierenden Agenz Harnstoff in einem Waschschritt nachgewiesen werden. Dabei betrug die Konzentration dieser Substanz 4 Mol. Die Testung erfolgte mit dem SERION ELISA *classic* Röteln IgG.

### Ergebnis siehe Tabelle 2:

4 molarer Harnstoff bewirkt bei einem pH-Wert von 9,0 noch keine gute Diskriminierung zwischen hochaviden und niedrigaviden Seren. Wird der pH-Wert auf einen Bereich zwischen 11 und 12 eingestellt, bewirkt 4 molarer Harnstoff eine sehr gute Unterscheidung zwischen hochaviden und niedrigaviden Seren bei Probanden mit einer Immunität gegen Röteln.

### Beispiel 3

Es sollte nachgewiesen werden, dass der pH-Effekt nicht nur bei Verwendung von Hamstofflösung eine entscheidende Rolle spielt, sondern unabhängig von der jeweiligen Substanz im Waschschritt allgemein wirksam ist. Zum Nachweis der allgemeinen Wirkung eines unüblichen pH-Wertes auf die Aviditätsbestimmung wurde ein weiteres proteindenaturierendes Agenz eingesetzt. Es sollte unter Verwendung von Guanidin-Hydrochlorid eine Aviditätsprüfung der Seren von Patienten mit frischer oder lang zurückliegender Infektion durch das Röteln Virus mittels ELISA erfolgen. Dabei betrug die Konzentration dieser Substanz 1 Mol. Die Testung erfolgte mit dem SERION ELISA *classic* Röteln IgG.

### Ergebnis siehe Tabelle 3:

1 molares Guanidin-Hydrochlorid bewirkt im neutralen pH-Bereich keine gute Diskriminierung zwischen hochaviden und niedrigaviden Seren. Wird der pH-Wert auf einen Bereich zwischen 3 und 2,5 eingestellt, bewirkt 1 molares Guanidin-Hydrochlorid eine sehr gute Unterscheidung zwischen hochaviden und niedrigaviden Seren bei Probanden mit einer Immunität gegen Röteln.

### Beispiel 4

Es sollte nachgewiesen werden, dass der pH-Effekt bei der Aviditätsbestimmung nicht nur bei Probanden mit einer Immunität gegen Röteln eine entscheidende Rolle spielt, sondern unabhängig vom jeweiligen Erreger allgemein wirksam ist.

Die Wirkung eines unüblichen pH-Wertes auf die Aviditätsbestimmung der Seren von Patienten mit frischer oder lang zurückliegender Infektion durch das Epstein Barr Virus mittels ELISA sollte unter Verwendung des bekannten proteindenaturierenden Agenz Harnstoff in einem Waschschritt nachgewiesen werden. Dabei betrug die Konzentration dieser Substanz 4 Mol. Die Testung erfolgte mit dem SERION ELISA *classic* EBV-VCA IgG.

### Ergebnis siehe Tabelle 4:

4 molarer Harnstoff bewirkt im neutralen pH-Bereich keine gute Diskriminierung zwischen hochaviden und niedrigaviden Seren. Wird der pH-Wert auf einen Wert von ca 4,0 eingestellt, bewirkt 4 molarer Harnstoff eine sehr gute Unterscheidung zwischen hochaviden und niedrigaviden Seren bei Probanden mit einer Immunität gegen EBV.

## Patentansprüche

1. Verfahren zum qualitativen oder quantitativen Nachweis eines Antikörpers, worin dieser Antikörper mit dem Antigen, gegen welches er gerichtet ist, in Kontakt gebracht wird, so dass sich Immunkomplexe bilden können, und worin der Reaktionsansatz mit einem proteindenaturierenden Agens in Kontakt gebracht wird, welches Immunkompfexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen bestimmt wird,
**dadurch gekennzeichnet,**
**dass** die eingesetzten proteindenaturierenden Agenzien auf einen pH-Wert eingestellt werden, der außerhalb des für immunologische Teste üblichen pH-Bereiches von 5,0 bis 9,0 liegt.

2. Verfahren zur Bestimmung der Avidität eines Antikörpers, worin der Antikörper in einem ersten und einem zweiten Ansatz unabhängig voneinander mit dem Antigen, gegen welches der Antikörper gerichtet ist, in Kontakt gebracht wird, so dass sich Immunkomplexe bilden können, und worin einer der beiden Ansätze mit einem proteindenaturierenden Agens in Kontakt gebracht wird, welches Immunkomplexe enthaltend Antikörper niedriger Avidität destabilisiert, während Immunkomplexe enthaltend Antikörper höherer Avidität weitgehend erhalten bleiben, und worin das Ausmaß der Bindung des Antikörpers mit dem Antigen in beiden Proben unabhängig voneinander nach einem dem Fachmann bekannten Verfahren bestimmt wird, und wobei sich die Avidität des Antikörpers aus dem Verhältnis des Ausmaßes der Antigen-Antikörperbindungen des ersten und des zweiten Ansatzes ergibt, **dadurch gekennzeichnet, dass** die eingesetzten proteindenaturierenden Agenzien auf einen pH-Wert eingestellt werden, der außerhalb des für immunologische Teste üblichen pH-Bereiches von 5,0 bis 9,0 liegt.

3. Verfahren gemäß Anspruch 1 oder 2, worin das Antigen in festphasengebundener Form mit dem Antikörper in Kontakt gebracht und anschließend mit irgendeiner Pufferlösung gleich welcher Zusammensetzung gewaschen wird, **dadurch gekennzeichnet, dass** diese Pufferlösung auf einen pH-Wert eingestellt wurde, der außerhalb des für immunologische Teste üblichen pH-Bereiches von 5,0 bis 9,0 liegt.

4. Verfahren gemäß einem der vorhergehenden Ansprüche, **dadurch gekennzeichnet, dass** es als Enzymimmuntest, Radioimmuntest, Westem Blot oder Immunfluoreszenztest ausgeführt wird.

5. Verfahren gemäß einem der vorhergehenden Ansprüche, **dadurch gekennzeichnet, dass** es zum Nachweis diagnostisch relevanter Antikörper in Körperflüssigkeiten verwendet wird.

6. Verfahren gemäß Anspruch 5, **dadurch gekennzeichnet, dass** die diagnostisch relevanten Antikörper gegen Viren, Bakterien oder Parasiten gerichtet sind.

7. Verfahren gemäß Anspruch 5 oder 6, **dadurch gekennzeichnet, dass** die diagnostisch relevanten Antikörper gegen EBV, Rötelnvirus, CMV, Hantavirus, Parvovirus B19, VZV, HHV 6, HBV, HCV, HIV, RSV, HSV 1, HSV 2 oder Toxoplasma gondii gerichtet sind.

8. Verfahren zur Differenzierung früher Infektionen von älteren Infektionen durch Bestimmung der Antikörperavidität nach einem Verfahren gemäß einem der vorhergehenden Ansprüche.

9. Verfahren zum Nachweis einer akuten oder kürzlich abgelaufenen Rötelnvirusinfektion, CMV-Erstinfektion, EBV-Erstinfektion, HSV-Erstinfektion oder Toxoplasma gondii-Infektion.

## Claims

1. Process for the qualitative or quantitative detection of an antibody, which comprises contacting the said antibody with the antigen against which it is directed so as to enable immunocomplexes to form, and which comprises contacting the reaction mixture with a protein-denaturating agent which destabilizes immunocomplexes comprising antibodies of low avidity, while immunocomplexes comprising antibodies of higher avidity substantially remain intact, and which comprises determining the extent of binding of the antibody with the antigen,
**characterized in that**
the protein-denaturating agents used are adjusted to a pH which lies outside the pH range from 5.0 to 9.0 customary for immunological assays.

2. Process for determining the avidity of an antibody, which comprises contacting the antibody in a first and a second reaction mixture independently of one another with the antigen against which the antibody is directed so as to enable immunocomplexes to form, and which comprises contacting one of the two reaction mixtures with a protein-denaturating agent which destabilizes immunocomplexes comprising antibodies of low avidity, while immunocomplexes comprising antibodies of higher avidity substantially remain intact, and which comprises determining the extent of binding of the antibody with the antigen in both samples independently of one another by a process known to the skilled worker, with the avidity of the antibody resulting from the ratio of the extent of antigen-antibody binding of the first and second reaction mixtures, **characterized in that** the protein-denaturating agents used are adjusted to a pH which lies outside the pH range from 5.0 to 9.0 customary for immunological assays.

3. Process according to Claim 1 or 2, which comprises contacting the solid phase-bound antigen with the antibody, followed by washing with any buffer solution of any composition, **characterized in that** this buffer solution has been adjusted to a pH which lies outside the pH range from 5.0 to 9.0 customary for immunological assays.

4. Process according to any of the preceding claims, **characterized in that** it is carried out as an enzyme immunoassay, radioimmunoassay, Western blot or immunofluorescence assay.

5. Process according to any of the preceding claims, **characterized in that** it is used for detecting diagnostically relevant antibodies in body fluids.

6. Process according to Claim 5, **characterized in that** the diagnostically relevant antibodies are directed against viruses, bacteria or parasites.

7. Process according to Claim 5 or 6, **characterized in that** the diagnostically relevant antibodies are directed against EBV, rubella virus, CMV, hantavirus, parvovirus B19, VZV, HHV 6, HBV, HCV, HIV, RSV, HSV 1, HSV 2 or Toxoplasma gondii.

8. Process for differentiating early infections from older infections by determining the antibody avidity by a process according to any of the preceding claims.

9. Process for the detection of an acute or recent rubella virus infection, a first CMV infection, first EBV infection, first HSV infection or Toxoplasma gondii infection.

## Revendications

1. Procédé pour déceler qualitativement ou quantitativement un anticorps, dans lequel cet anticorps est mis en contact avec l'antigène contre lequel il est dirigé de façon à pouvoir former des immuns-complexes et dans lequel la masse réactionnelle est amenée en contact avec un agent de dénaturation de protéine qui déstabilise des immuns-complexes contenant des anticorps de faible avidité tandis que des immuns-complexes contenant des anticorps d'avidité supérieure sont largement conservés, et dans lequel la mesure de la liaison de l'anticorps à l'antigène est déterminée,
**caractérisé en ce que**
les agents de dénaturation de protéine mis en oeuvre sont ajustés à une valeur de pH qui est située en dehors de la gamme de pH habituelle pour des tests immunologiques de 5,0 à 9,0.

2. Procédé de détermination de l'avidité d'un anticorps, dans lequel l'anticorps est, dans une première masse et dans une deuxième masse, amené indépendamment l'une de l'autre en contact avec l'antigène contre lequel l'anticorps est dirigé de façon à pouvoir former des immuns-complexes et dans lequel une des deux masses est amenée en contact avec un agent de dénaturation de protéine qui déstabilise des immuns-complexes contenant des anticorps de faible avidité tandis que des immuns-complexes contenant des anticorps d'avidité supérieure sont largement conservés et dans lequel la mesure de la liaison de l'anticorps à l'antigène est déterminée dans les deux échantillons indépendamment l'un de l'autre selon un processus connu de l'homme de métier, l'avidité de l'anticorps étant obtenue du rapport entre les mesures des liaisons antigène-anticorps de la première masse et de la deuxième masse, **caractérisé en ce que** les agents de dénaturation de protéine mis en oeuvre sont ajustés à une valeur de pH qui est située en dehors de la gamme de pH courante pour des tests immunologiques de 5,0 à 9,0.

3. Procédé suivant la revendication 1 ou 2, dans lequel l'antigène est amené, sous une forme liée à une phase solide, en contact avec l'anticorps et ensuite est lavé avec une quelconque solution tampon également de n'importe quelle composition, **caractérisé en ce que** cette solution tampon a été ajustée à une valeur de pH qui est située en dehors de la gamme de pH courante pour des tests immunologiques de 5,0 à 9,0.

4. Procédé suivant l'une des revendications précédentes, **caractérisé en ce qu'**il est réalisé sous la forme d'un essai immunologique enzymatique, d'un essai radio-immunologique, d'un western-blot ou d'un essai d'immunofluorescence.

5. Procédé suivant l'une des revendications précédentes, **caractérisé en ce qu'**il est utilisé pour déceler des anticorps significatifs du point de vue du diagnostic dans des liquides corporels.

6. Procédé suivant la revendication 5, **caractérisé en ce que** les anticorps significatifs du point de vue du diagnostic sont dirigés contre des virus, des bactéries ou des parasites.

7. Procédé suivant la revendication 5 ou 6,
**caractérisé en ce que** les anticorps significatifs du point de vue du diagnostic sont dirigés contre les EBV, virus de la rubéole, CMV, Hantavirus, Parvovirus, B19, VZV, HHV 6, HBV, HCV, HIV, RSV, HSV 1, HSV 2 ou Toxoplasma gondii.

8. Procédé de différenciation d'infections précoces par rapport à des infections anciennes, par détermination de l'avidité d'anticorps selon un procédé suivant l'une des revendications précédentes.

9. Procédé pour déceler une infection par le virus de la rubéole, une primo-infection par CMV, une primo-infection par EBV, une primo-infection par HSV ou une infection par Toxoplasma gondii qui s'est déroulée de manière aiguë ou brève.
